Europäisches Patentamt

European Patent Office

Office européen des brevets

(19)

(11) Publication number: **0 218 751**
**B1**

## EUROPEAN PATENT SPECIFICATION

(12)

(45) Date of publication of the patent specification:
**12.09.90**

(51) Int. Cl.⁵: **H04N 13/00**

(21) Application number: **85113213.4**

(22) Date of filing: **17.10.85**

(54) Stereoscopic remote viewing system.

(43) Date of publication of application:
**22.04.87 Bulletin 87/17**

(45) Publication of the grant of the patent:
**12.09.90 Bulletin 90/37**

(84) Designated Contracting States:
**BE CH DE FR GB IT LI NL**

(56) References cited:
**US-A- 2 955 156**
**US-A- 4 395 731**
**US-A- 4 559 555**

**PROCEEDINGS OF THE S.I.D., vol. 24, no. 1, 1983, pages 73-80, Los Angeles, US; R.L. PEPPER et al.: "The influence of camera separation and head movement on perceptual performance under direct and TV-displayed conditions"**
**F&KT - Fernseh-und Kinotechnik, Vol. 36, Nr. 11/82, S. 441-445**

(73) Proprietor: **Schoolman, Arnold, 6420 Prospect, Kansas City Missouri(US)**

(72) Inventor: **Schoolman, Arnold, 6420 Prospect, Kansas City Missouri(US)**

(74) Representative: **Baillie, Iain Cameron et al, c/o Ladas & Parry Isartorplatz 5, D-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to remote video systems and, more particularly, to a stereoscopic video system with a head worn display for use as in the control of an industrial robot.

Certain manufacturing operations lend themselves to performance by manually controlled industrial robots because of the hazardous nature of such operations. The hazards arise from the materials involved such as explosive, corrosive, pathogenic, cryogenic, radioactive or the like materials or from the necessity of performing such operations in atmospheres which are poisonous or non-life supporting to humans such as welding in inert gases or operations which require a vacuum. Other situations which benefit from manually controlled robotics include operations which are necessarily carried out by remote control such as some deep sea operations and outer space operations. While some such operations can be performed by automatically controlled robots with feedback loops for selfcorrection, others require direct human control.

The primary human feedback senses for manual activities are usually vision and touch or feel. considerable effort has been expended in the development of remote controls which have a useful feel such that a given amount of manual movement of a control results in an expected result. A portion of the success of such a control involves learning to use the control by the operator.

The development of "remote sight" has, to an extent, lagged the development of remote manipulation because of the complexities of human vision and the complexities of the systems for aiding human vision, namely, television. Human vision is stereoscopic; that is, the right and left eyes sense slightly different images because of the lateral separation therebetween. The brain interprets the difference in image to derive depth perception such that a three dimensional visual sense results. Through practice as an individual ages, eye-hand coordination can become quite accurate.

Television has for the most part been a two dimensional medium, notwithstanding such techniques in color television as so-called "3D" effects wherein two of the component colors, such as red and green, are offset laterally and when viewed through a set of properly oriented red and green spectacle lenses gives an approximation of three dimensional vision. Such effects, while arguably adequate for applications as in motion pictures, do not convey an accurate enough sense of depth for such activities as the control of a robotic arm. In order to obtain a truer video image of the depth dimension, that is in the direction of the line of sight of a video camera, a second video camera which is laterally spaced or elevated from the first camera has been required. A viewer is then required to watch and coordinate the images on two separate viewing screens.

The result is an improvement over two dimensional viewing, but does not provide the apparent simplicity and the convenience of natural vision.

In more recent years, image (video camera) tubes and picture tubes, or cathode ray tubes (CRT's), have been miniaturized to the extent that a pair of image tubes can be positioned to approximate the perspective of the human eyes; and a pair of CRT's can be positioned and the images projected preferably indirectly, to the eyes of a human such that a good approximation of natural stereoscopic vision can be achieved. Non-CRT visual display devices such as liquid crystal, light emitting diode, and fluorescent matrix displays are currently being applied to reduce the size of video displays even further.

The article appearing in volume 36 (No. 11/1982: pages 441–445) of F & KT – Fernseh- und Kino-Technik is concerned with manipulation and orientation of sensing cameras and general view of images produced thereby.

According to the present invention there is provided a stereoscopic remote viewing system including first and second image sensors spaced apart to provide a left video signal and a right video signal, said video signals representing scenes as sensed by said image sensors, a platform supporting said sensors for convergence of lines of sight of said sensors substantially at a subject to be viewed, a movable support for moving said platform in three dimensions selectively to aim said sensors, and a motor for moving said platform support and a control associated with said motor, characterized by a head unit adapted for wearing on the head of a viewer, a first video display device directly linked with said first image sensor, a second video display device directly linked with said second image sensor respectively to receive said left and right video signals and display images which are reproductions of scenes sensed by said image sensors, said first and second video display devices being mounted on said head unit and cooperating with said first and second image sensors to provide a natural vision stereoscopic image to the viewer.

The present invention provides apparatus for stereoscopically viewing a remote subject. The apparatus includes a pair of video cameras mounted on a platform which is capable of movement in three dimensions. The cameras include motors for focus and zoom control and convergence motors such that the lines of sight of the cameras coincide at the focal point of the cameras. The camera platform is mounted on a semicircular track and is movable therealong for azimuth aiming of the cameras. The track is rotatable about a diametric axis for elevation of the cameras.

The cameras are coupled to a head worn video display unit including means such as a helmet with a pair of video display devices such as miniature cathode ray tubes or picture tubes and optical and mirror or prismatic elemtens to project the displayed images into focus in the eyes of a viewer wearing the video display. The helmet also includes circuitry for generating sweep signals for the CRT's and circuits for processing the video signals from the cameras.

A computer system is connected to the stereoscopic display through a video source selector for the display of reference texts and graphics related to the operation to be performed by a robotic device associated with the viewing system. For displaying texts and graphics which do not require a stereoscopic display, the computer is controlled to feed the same image to the right and left video channels for a binocular monoscopic view of such information.

The principal objects of the present invention are: to provide an improved apparatus for remotely viewing manually controlled operations; to provide such an apparatus which facilitates the remote operation of a robotic arm and hand device; to provide such an apparatus which provides a three dimensional or in view of a remote operation; to provide such an apparatus including a pair of movably mounted video cameras and a corresponding pair of video displays adapted as a head worn stereoscopic unit for placement on the head of a viewer to project a pair of images from the cameras into the eyes of the viewer; to provide such an apparatus wherein the cameras are mounted on a bracket or platform which is positioned on a semicircular track for movement along the track for azimuth aiming of the cameras, the semicircular track being rotatable about a horizontal diametric axis for elevation aiming of the cameras; to provide such an apparatus including controls for focusing the cameras and zooming of the cameras to change perspective and viewed size of the subject; to provide such an apparatus including means for maintaining the focus and line of sight convergence of the cameras during zooming; to provide such an apparatus including a computer selectively connected to the stereoscopic display unit for the display of texts and graphic information related to the operation performed during use of the viewing system; and to provide such a stereoscopic viewing system which is economical to manufacture, convenient in operation, and which is particularly well adapted for its intended purpose.

Other objects and advantages of this invention will become apparent from the following description taken in conjunction with the accompanying drawings 5 wherein are set forth, by way of illustration and example, certain embodiments of this invention.

The drawings constitute a part of this specification and include exemplary embodiments of the present invention and illustrate various objects and features thereof.

Fig. 1 is a diagrammatic plan view of a stereoscopic remote viewing system according to the present invention, shown in association with a manually controlled robotic device.

Fig. 2 is a side elevational view of a movable camera platform support of the system.

Fig. 3 is an enlarged fragmentary plan view of the video camera platform.

Fig. 4 is a further enlarged sectional view taken on line 4–4 of Fig. 3 and illustrates details of the video camera unit.

Fig. 5 is a side elevational view of a stereoscopic display unit of the system with portions broken away to illustrate the optical elements which project a pair of images into the eyes of the viewer.

Fig. 6 is a front elevational view of the display unit of the system.

Fig. 7 is a top plan view of the display unit with portions broken away to illustrate further details of the projection optics.

Fig. 8 is a block diagram illustrating the functional relationships of a computer system for storing and recalling texts and graphics related to the operation performed and viewed through the viewing system, a pair of video cameras, and the display unit.

Fig. 9 is a block diagram illustrating controls for operation of the camera unit of the viewing system.

As required, detailed embodiments of the present invention are disclosed herein; however, it is to be understood that the disclosed embodiments are merely exemplary of the invention which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

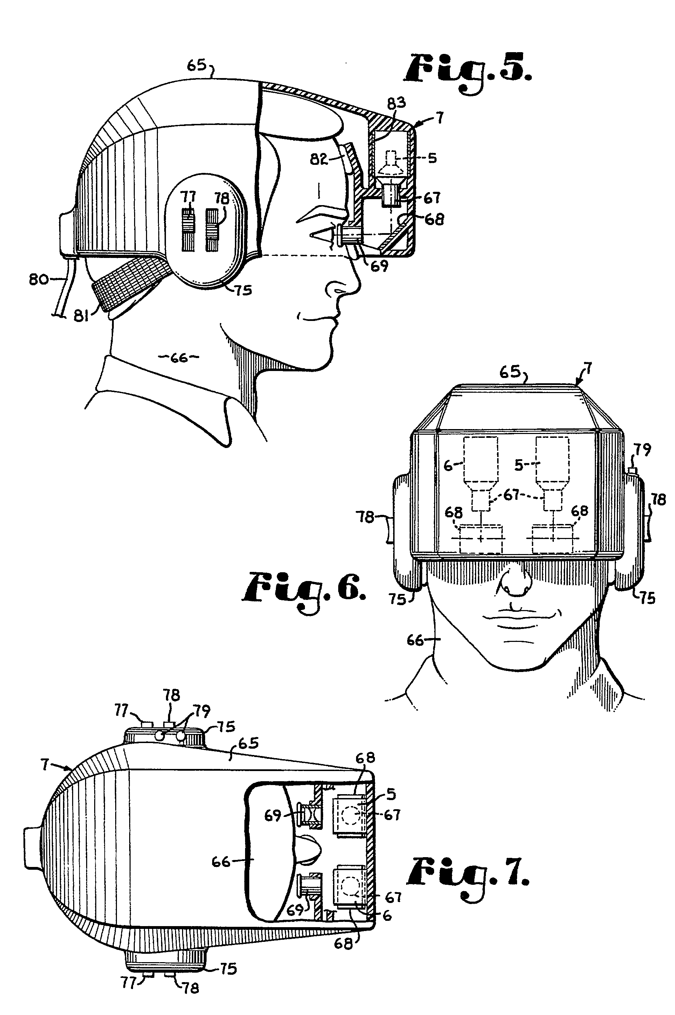

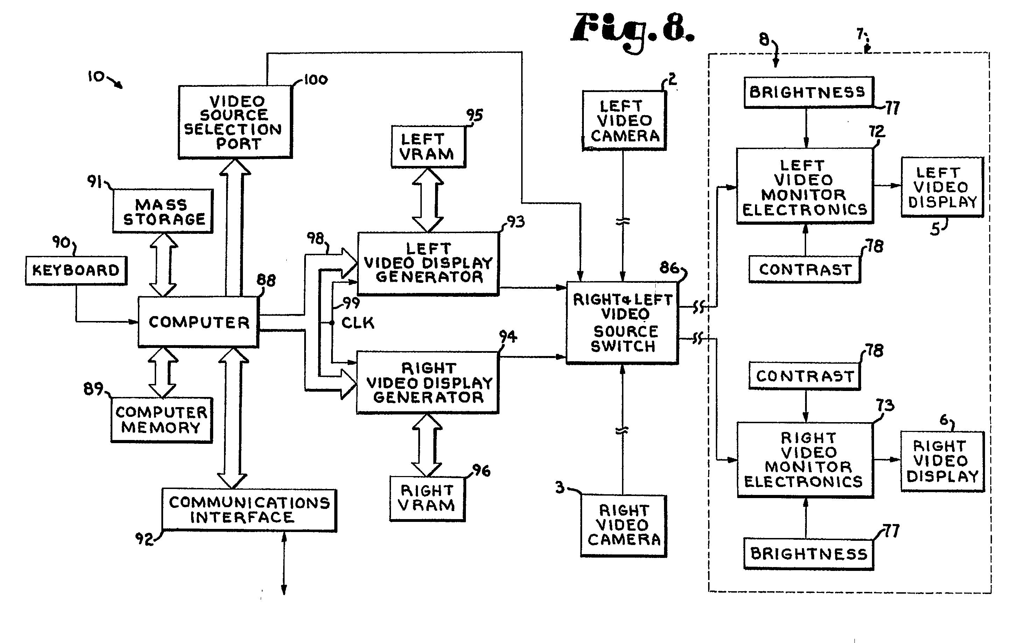

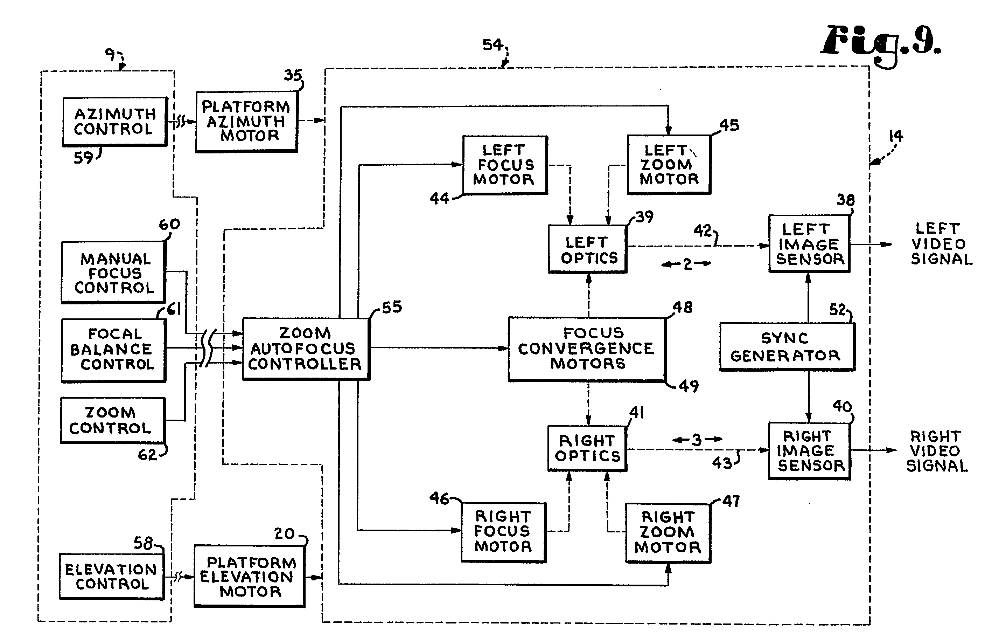

The reference numeral 1 generally designates a stereoscopic remote viewing system according to the present invention. The system 1 generally includes a pair of video cameras 2 and 3 mounted on a three dimensionally movable camera aiming support 4 and a pair of video display devices 5 and 6 (Figs. 5–7) mounted in a video display unit 7 which is adapted to be worn on the head of a viewing individual. The display unit 7 includes video circuitry 8 (Fig. 8) to adapt the display devices 5 and 6 as video monitors for viewing the images sensed by the cameras 2 and 3. The cameras 2 and 3 are positioned to capture a stereoscopic view of a scene; and similarly the display devices 5 and 6 are positioned to provide a stereoscopic display of the reproduced images for the viewer. A system control console 9 allows the viewer to control the position and the optical features of the camera 2 and 3. A computer system 10 is selectively connected to the video display unit 7 and is employed to store and retrieve digitally encoded texts and graphics for display on the video display unit 7. The system 1 is particularly adapted for remotely viewing operations such as the operations of a remotely controlled robotic device 110.

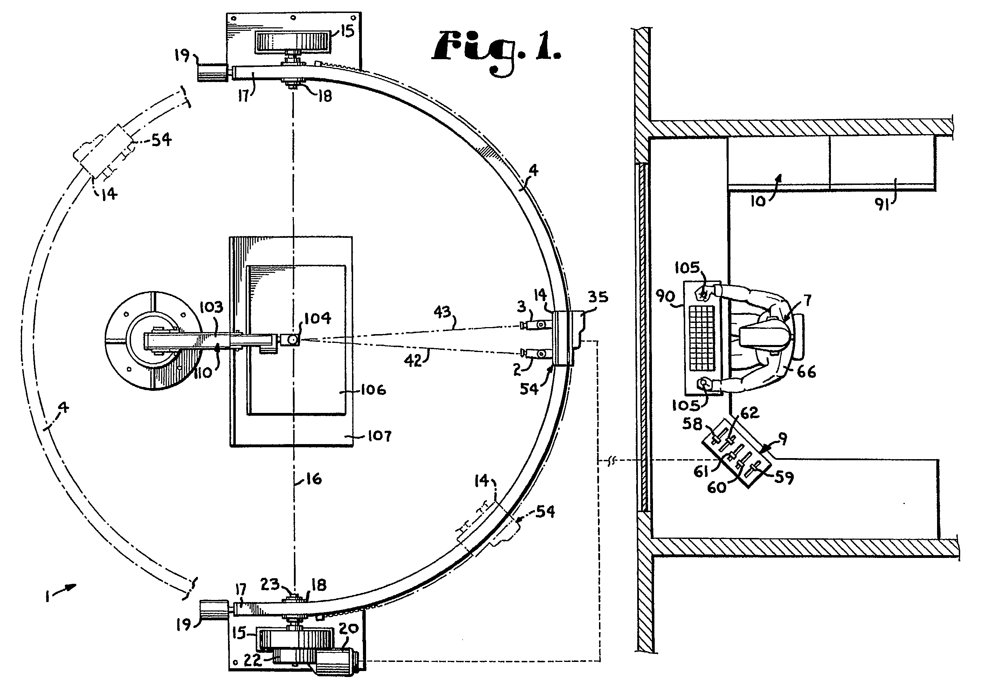

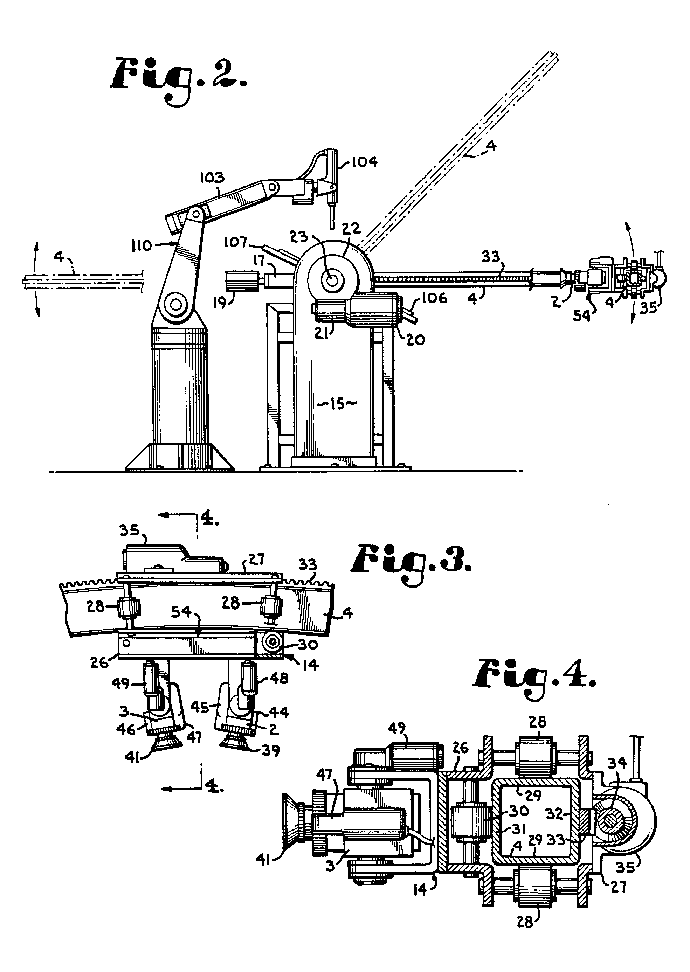

Referring to Fig. 3, the cameras 2 and 3 are mounted in spaced relation on a camera bracket or platform 14 which is, in turn, mounted for movement along the camera aiming support 4. The support 4 is a semicircular guide track which is pivotally connected at extremities of the track 4 to track supports 15 for rotation of the track 4 about a horizontal diametric axis 16 of the track 4. The illustrated track 4 has square tubular cross section. The extremities 17 of the track 4 extend past the pivot joints 18 which connect the track 4 to the supports 15. The extremities 17 preferably have counterweights 19 thereon to counterbalance the track structure on the pivot joints 18. An elevation motor 20 is mounted on one of the track supports 15 and operatively engages the track 4 to pivot same for elevation aiming of the cameras 2 and 3. For example, a helical or worm gear in a housing 21 is positioned on a rotary shaft of the motor 20 and engages a pinion gear in a housing 22 and fixed on a pivot shaft 23 of the track 4. The degree of angular movement of the track 4 which is allowable or desired is determined by the dimensions of the track 4 in comparison to the height of the supports 15 and the degree of coverage of the operation required. Preferably, the track 4 is able to cover at least a hemisphere.

The camera platform 14 may be any structure which supports the cameras 2 and 3 for movement along the track 4. The platform 14 illustrated in Fig. 4 includes an inner bracket 26 and an outer bracket 27 joined by sets of opposed side rollers 28 which contact side walls 29 of the track 4. An inner set of rollers 30 engages an inside wall 31 of the track 4. The outer wall 32 has an elongated rack gear 33 positioned on the outer surface thereof. The gear 33 cooperates with a helical or worm gear 34 which is affixed to the rotary shaft of an azimuth motor 35 which is mounted on the outer bracket 27. Rotation of the worm gear 34 causes the platform 14 to move along the track 4 for azimuth aiming of the cameras 2 and 3. The rollers 28 and 30 are preferably resiliently urged against the respective walls of the track 4 such that the platform 14 is securely held on the track 4.

Referring to Figs. 3, 4, and 9, the left hand camera 2 includes a left image sensor 38 and left camera optics 39 while the right camera 3 includes a right image sensor 40 and right camera optics 41. The image sensors 38 and 40 may be any type of conventional video image sensors including solid state image sensors. The optics 39 and 41 are mounted in the cameras 2 and 3 for movement along respective lines of sight or optical axes 42 or 43 to focus an image on the associated image sensor and to change the optical perspective of the image transmitted to the associated image sensor. For these purposes, the left video camera 2 includes a left focus motor 44 and a left zoom motor 45 while the right video camera 3 includes a right focus motor 46 and a right zoom motor 47. In order for a useful stereoscopic image to be sensed by the pair of cameras 2 and 3, it is necessary for the cameras to be aimed such that the scenes sensed by the cameras 2 and 3 substantially overlap, that is, that the focal points of the cameras 2 and 3 substantially coincide. Therefore, the cameras 2 and 3 include focus convergence motors 48 and 49 respectively. The cameras 2 and 3 are mounted for rotation substantially in the plane of the platform track 4 and have the motors 48 and 49 connecting the associated cameras to the inner bracket 26 of the camera platform 14. The focus convergence motors 48 and 49 are activated to maintain the coincidence of the focal points of the cameras 2 and 3 as the focal points of the cameras are changed by operation of the focus motors 44 and 46.

In order to prevent undesired visual effects between the left and right video channels, the left and right image sensors 38 and 40 are provided with common sync signals provided by a sync generator 52 such that the scans of the left and right image sensors 38 and 40 are synchronized. Preferably, the cameras 2 and 3 include means for maintaining focused images as the optics 39 and 41 are zoomed. Mechanical arrangements for maintaining focus during zooming are known and may be employed in the system 1. As illustrated in Fig. 9, the camera unit 54 includes a zoom autofocus controller 55 which is a dedicated control computer interconnected with the focus motors 44 and 46, the zoom motors 45 and 47, and the focus convergence motors 48 and 49. Autofocus controller arrangements for monoscopic lens systems are known. The autofocus controller 55 is adapted for maintaining the focus during zooming for the left and right cameras 2 and 3, and, further, controls the convergence of the focal points of the cameras 2 and 3. Although not detailed in Fig. 9, sensors would be required for determining the positions of the left and right optics 39 and 41 for the generation of autofocus control signals. Such sensing could include shaft encoders of various descriptions positioned on the shafts of the motors of the camera unit 54. The details of such position sensors are believed to be within the capabilities of one skilled in the digital control of electric motors.

The viewing system control panel 9 includes an elevation control 58 connected to the platform elevation motor 20 and an azimuth control 59 connected to the platform azimuth motor 35. The manual focus control 60 is provided for fine adjustment of the focal positions of the left and right optics 39 and 41 in unison. A focal balance control 61 provides for differential adjustment of the focal positions of the optics 39 and 41 to compensate for the individual visual acuities of the user of the system 1. A zoom control 62 is operated to cause zooming of the left and right optics 39 and 41 in unison. The controls 60, 61, and 62 are operatively connected to the motors of the camera unit 54 through the zoom autofocus controller block 55.

Figs. 5–7 illustrate details of the head worn video display unit 7. The unit 7 includes a cap or helmet 65 in which the components of the unit 7 are mounted. The illustrated video display devices 5 and 6 are CRT's and are mounted within the helmet 65 for indirect projection of the images therefrom into the eyes of the viewer 66 in order to decrease the possibility of projecting X-rays into the eyes of the viewer. The use of solid state matrix displays instead of the CRT's 5 and 6 is contemplated such that the system 1 is not to be limited to the use of CRT's as video display devices. The CRT's 5 and 6 are mounted for downward projection of the images thereof through projection optical elements 67 toward right angle reflecting elements 68 such as mirrors or prisms and from there through viewing lenses 69 to the eyes of the viewer 66. The viewing lenses 69 are provided to compensate for the short focal distance between the eyes of the viewer 66 and the display devices 5 and 6 such that the images can be focused at a greater effective distance. The viewing lenses 69 are preferably finely adjustable to accommodate the visual capabilities of the eyes of the viewer 66. Further, the lateral positions of the right and left video display components are preferably adjustable to accommodate the spacing between the eyes of the viewer 66, although such adjustment means are not illustrated.

Currently, color cathode ray tubes are not small enough for convenient use in the video display unit of the system 1. Therefore, using CRT's, the system 1 is restricted to monochrome or black and white technology. However, color liquid crystal matrix displays are known such that in general, the system 1 is not intended to be restricted to black and white viewing. With reference to Fig. 8, the components of the video display unit 7 comprise a head worn stereoscopic video monitor unit. Therefore, the left and right video channels include respective left and right video monitor circuitry 72 and 73. The monitor circuits 72 and 73 include sweep generators for generating the rasters on the displays 5 and 6 and further include video signal demultiplexers for separating sync and luminance signals from composite signals if such composite signals are employed, and conventional video circuits for processing and displaying the images represented by the video signals. If color technology is employed in the system 1, it is foreseen that the color component signals may either be provided as right and left color composite signals or as separated red, green, and blue (RGB) sets of color signals. The left and right monitor circuits are conventional and are configured according to the requirements of the system. It is preferred that the circuits 72 and 73 be mounted within the helmet 65 such as within ear bosses 75 or in an enlargement such as at the rear of the helmet 65 (not shown) to provide better balance of the helmet 65. Preferably, each of the circuits 72 and 73 includes at least a brightness control 77 and a contrast control 78 for adjustment of the displayed images. If color is provided in the system 1, color level and tint or individual RGB color controls (not shown) would appropriately be provided. In addition, an on/off-power switch 79 is provided for activating and deactivating the components within the video display unit 7.

The video signals from the cameras 2 and 3 are provided to the components within the helmet 65 as by a cable 80 which conveniently enters the video display unit 7 at the rear of the helmet 65. Although not illustrated, the helmet 65 preferably includes an adjustable harness for fitting the helmet to the head of the viewer 66. Means such as an elastic band 81 may be provided to retain the helmet in position on the head of the viewer. A forehead pad 82 is provided to maintain the position of the optical elements of the display unit 7 in relation to the eyes of the viewer. X-Ray shields 83 are preferably positioned to enclose the CRT's 5 and 6, particularly if color CRT's are employed.

The video signals from the left and right video cameras 2 and 3 are provided to the video display unit 7 through a right and left video source switch 86. The video source switch 86 also has the video output from the computer system 10 connected thereto whereby the video display unit 7 may be driven either by the computer system 10 or the video cameras 2 and 3. The video source switch 86 may include conventional switch components or coaxial switch components which are properly sequenced to isolate one video source from another.

Preferably, the video source switch components are operated by solenoid means (not shown) under control of the computer system 10. The switch 86 may be further expanded to provide for selection of other video sources such as video tape recorders, video discs, or television tuners (not shown). Further, it is foreseen that the video source switch 86 could be configured to allow the routing of video signals from the cameras 2 and 3 to the video display unit 7 and, additionally, to video recording devices to record the images as viewed for later analysis or for digitization and storage within a computer system such as the computer system 10.

The computer system 10 is, for the most part, a conventional general purpose digital computer. The illustrated computer system 10 includes a central processing unit or main computer board 88, a main computer memory 89, a keyboard 90, mass storage devices such as discs 91, and a communications interface 92 for communication between the computer system 10 and another computer. Normally, computers, particularly microcomputers, only require a single video channel for displaying text or graphics on a video monitor associated therewith. However, since the capability for stereoscopically displaying digitized graphic information is desired, the computer system 10 has left and right video channels interfaced thereto. The left and right hand video channels include respectively a left and a right video display generator 93 and 94. The video display generators 93 and 94 include associated video memories or video random access read/write memories (VRAM) 95 and 96. In general, the video display generators derive respective analog video signals from the digital information stored within the video memories 95 and 96. Further, the generators 93 and 94 control the flow of digital data from the computer 88 to the video memories 95 and 96. The video display generators 93 and 94 are essentially conventional and are preferably capable of high resolution displays. The generators 93 and 94 include respective address decoders for the proper routing of video information from the computer board 88 to the generators 93 and 94 over a video bus 98 which is interfaced to the computer board 88 through a video port. In order to synchronize the left and right video displays 5 and 6 when the computer video channels are selected, the right and left video display generators 93 and 94 are connected to a common clock line 99 of the video bus 98. The video source switch 86 receives a selection command from a video source selection port 100 which is interfaced to the computer board 88. The operator or viewer 66 selects the desired video source by operation of a key or keys on the keyboard 90.

The computer system 10 includes software for operation of the video source switch 86 and for storing, retrieving, and routing digitized images from the mass storage device 91 to the video display unit 7. The digitized images provided by the computer system 10 may be either stereoscopic or monoscopic whereas text which is displayed on the unit 7 need only be monoscopic. For monoscopic displays, the computer 88 feeds the same display data to the left and right display generators 93 and 94. For stereoscopic displays, different video data is routed to the left and right display generators 93 and 94. The computer system 10 may be programmed to display a menu upon the operation of a selected key on the keyboard 90 for the selection of functions related to the system 1 or may be programmed to respond to specific spelled-out commands.

The viewing system 1 is particularly well adapted for remotely viewing an operation such as detailed manufacturing operations performed by a manually controlled robotic device. The robotic device 110 illustrated in Figs. 1 and 2 includes a robotic arm 103 having a tool 104 attached thereto. The robotic device 110 is controlled as by the manipulation of robot control levers 105 which may be associated with either the control console 9 or the keyboard 90. The robotic arm 103 is controlled to selectively position the tool 104 for operations on a workpiece 106 positioned on a workpiece support 107. During the operation, the camera unit 54 may be placed anywhere along the platform guide track 4 and the track pivoted about its axis 16 to provide the best view to the operator 66. The stereoscopic images provided by the system 1 greatly facilitates the remote placement of the tool 104 in three dimensional space with respect to the workpiece 106. The computer system 10 provides for the display of texts such as instructions or procedures related to the operations performed by the robotic device 110 and viewed by the viewing system 1. Further, diagrammatic information and three dimensional graphic information can be recalled by the computer system 10 from the mass storage device 91 and displayed on the video display unit 7 for viewing by the operator 66. Such graphic information is intended to relate to the operations performed with the robotic device 110 and may include patterns for comparison with the workpiece being operated upon, views of a finished product, and the like.

## Claims

1. A stereoscopic remote viewing system including first and second image sensors (2, 3) spaced apart to provide a left video signal and a right video signal, said video signals representing scenes as sensed by said image sensors, a platform (14) supporting said sensors (2, 3) for convergence of lines of sight of said sensors substantially at a subject to be viewed, a movable support (4) for moving said platform in three dimensions selectively to aim said sensors, and a motor (20) for moving said platform support and a control associated with said motor, characterized by a head unit (7) adapted for wearing on the head of a viewer, a first video display device (5) directly linked with said first image sensor (2), a second vide display device (6) directly linked with said second image sensor (3) respectively to receive said left and right video signals and display images which are reproductions of scenes sensed by said image sensors, said first and second video display devices being mounted on said head unit and cooperating with said first and second image sensors to provide a natural vision stereoscopic image to the viewer.

2. A stereoscopic remote viewing system according to claim 1, characterized in that a computer (10) is provided for the storage and retrieval of text and graphic information, and in that a video source selector (100) is provided for selectively connecting said video display devices (5, 6) to said computer (10) and said image sensors (2, 3).

3. A stereoscopic remote viewing system according to claim 1, characterized in that the video display devices (5, 6) are cathode ray tubes (CRT's), and in that the head unit includes optical elements (68) positioned to project images reproduced on said CRT's indirectly into the eyes of said viewer.

4. A stereoscopic remote viewing system according to claim 1, characterized in that each image sensor includes an image sensor (38, 40), optic means (39, 41) positioned in relation to said image sensor to define a line of sight of said image sensor, and a focus motor (44, 46) connected to said optic means for positioning said optic means to focus a scene at a focal point along said line of sight on said image sensor.

5. A stereoscopic remote viewing system according to claim 4, characterized in that each image sesor includes a zoom motor (45, 47) connected to said optic means to vary the optical perspective of said scene as focused on said image sensor.

6. A stereoscopic remote viewing system according to claim 5, characterized in that a convergence motor (48, 49) is connected between the image sensor (2, 3) and the platform (14) for movement of the image sensor to intersect the lines of sight of said image sensor for substantial coincidence of the focal points of said image sensor.

7. A stereoscopic remote viewing system according to claim 5, characterized in that an autofocus controller (55) is connected to the focus motors (44, 46) and the zoom motors (45, 47) to maintain the focus of said image sensors during the operation of said zoom motors.

8. A stereoscopic remote viewing system according to claim 4, characterized in that a focal balance control is operatively connected to the focus motors for the fine adjustment of the focus positions of said optic means to compensate for the individual visual acuities of the eyes of said viewer.

9. A stereoscopic remote viewing system according to claim 1, characterized in that the support includes a substantially semicircular platform track (4), a track support supporting said platform track for elevation pivoting about a substantially horizontal diametric axis (16) of said track, and in that the platform (14) is supported on said track for movement therealong.

10. A stereoscopic remote viewing system according to claim 9, characterized in that an azimuth motor (35) is engaged between the platform and the track for movement of said platform means along said track to motivate azimuth aiming of said platform, and in that an elevation motor (20) is engaged between said track and said track support to pivot said track about said diametric axis to motivate the elevation aiming of said platform.

11. A stereoscopic remote viewing system according to claim 10, characterized in that a rack gear (33) extends along said platform track (4), in that the azimuth motor (35) is mounted on said track support and has a rotary shaft, and in that a helical gear (34) is positioned on said shaft and engages said rack gear whereby rotation of said shaft urges said platform along said platform along said platform track.

12. A stereoscopic remote viewing system according to claim 10, characterized in that a pinion gear is affixed to said platform track (4), said gear having a gear axis postioned coaxially with said diametric axis of said track, in that the elevation motor (20) is mounted on said platform and has a rotary shaft, and in that a helical gear is positioned on said shaft and engages said pinion gear whereby rotation of said shaft causes the pivoting of said platform track about said diametric axis (16).

13. A stereoscopic remote viewing system according to claim 1, characterized in that a robotic tool (110) is manually controllable by a wearer of said head unit for viewing the operations performed by said tool with said viewing system.

14. A stereoscopic remote viewing system according to claim 1, characterized by a substantially semicircular platform track, a platform track support supporting said platform track for elevation pivoting about a horizontal diametric axis of said track, and platform being supported in said track for movement therealong and carrying camera sensors, an azimuth motor engaged between said platform and said track for movement of said platform along said track to pivot said track about said diametric axis to motivate the elevation aiming of said platform, and camera aiming control means connected to said azimuth and elevation motors for manipulation by a viewer using said viewing system to aim said camera platform.


**Patentansprüche**

1. Stereoskopisches Fernbetrachtungssystem mit einem ersten und einem zweiten Bildsensor (2, 3), die in einem solchen Abstand voneinander angeordnet sind, daß sie ein linkes und ein rechtes Videosignal erzeugen, die von den Bildsensoren erfaßte Szenen darstellen, mit einer Plattform (14), die die Sensoren (2, 3) derart trägt, daß die Sichtlinien der Sensoren im wesentlichen zu einem Betrachtungsgegenstand konvergieren, mit einem bewegbaren Träger (4) zum Einrichten der Bildsensoren durch wahlweises Bewegen der Plattform in drei Dimensionen, mit einem Motor (20) zum Bewegen des Plattformträgers und mit einer dem Motor zugeordneten Steuereinrichtung, gekennzeichnet durch eine Kopfeinheit (7), die ein Betrachter auf dem Kopf tragen kann, durch ein mit dem ersten Bildsensor (2) direkt verbundenes erstes Videosichtgerät (5) und durch ein mit dem zweiten Bildsensor (3) direkt verbundenes zweites Videosichtgerät (6), die dazu dienen, das linke bzw. das rechte Videosignal zu empfangen und Bilder darzustellen, die Wiedergaben von den Bildsensoren erfaßter Szenen sind, wobei das erste und das zweite Videosichtgerät auf der Kopfeinheit montiert sind und mit dem ersten und dem zweiten Bildsensor derart zusammenwirken, daß sie für den Betrachter ein durch natürliches Sehen zu betrachtendes, stereoskopisches Bild erzeugen.

2. Stereoskopisches Fernbetrachtungssystem nach Anspruch 1, dadurch gekennzeichnet, daß zum Speichern und Wiedergewinnen von Text und graphischer Information ein Computer (10) vorgesehen ist und daß zum wahlweisen Verbinden der Videosichtgeräte (5, 6) mit dem Computer (10) und mit den Bildsensoren (2, 3) ein Videosignalquellenwähler (100) vorgesehen ist.

3. Stereoskopisches Fernbetrachtungssystem nach Anspruch 1, dadurch gekennzeichnet, daß die Videosichtgeräte (5, 6) Kathodenstrahlröhren (CRT) sind und daß die Kopfeinheit optische Elemente (68) aufweist, die so angeordnet sind, daß sie von den Kathodenstrahlröhren wiedergegebene Bilder indirekt in die Augen des Betrachters projizieren.

4. Stereoskopisches Fernbetrachtungssystem nach Anspruch 1, dadurch gekennzeichnet, daß jeder Bildsensor einen Bildsensor (38, 40) besitzt, ferner optische Mittel (39, 41), die relativ zu dem Bildsensor so angeordnet sind, daß sie für den Bildsensor eine Sichtlinie definieren, und einen mit den optischen Mitteln verbundenen Fokussiermotor, der dazu dient, die optischen Mittel auf eine Szene an einem auf der Sichtlinie des Bildsensors liegenden Brennpunkt zu fokussieren.

5. Stereoskopisches Fernbetrachtungssystem nach Anspruch 4, dadurch gekennzeichnet, daß jeder Bildsensor einen mit den optischen Mitteln verbundenen Zoommotor (45, 47) zum Verändern der optischen Perspektive der auf den Bildsensor fokussierten Szene besitzt.

6. Stereoskopisches Fernbetrachtungssystem nach Anspruch 5, dadurch gekennzeichnet, daß die Bildsensoren (2, 3) mit der Plattform (14) durch einen Konvergenzmotor (48, 49) verbunden sind, der dazu dient, die Bildsensoren derart zu bewegen, daß die Sichtlinien der Bildsensoren einander derart schneiden, daß die Brennpunkte der Bildsensoren im wesentlichen übereinstimmen.

7. Stereoskopisches Fernbetrachtungssystem nach Anspruch 5, dadurch gekennzeichnet, daß mit den Fokussiermotoren (44, 46) und den Zoommotoren (45, 47) ein Autofokus-Steuergerät (55) verbunden ist, das zum Aufrechterhalten der Fokussierung der Bildsensoren während des Betriebes der Zoommotoren dient.

8. Stereoskopisches Fernbetrachtungssystem nach Anspruch 4, dadurch gekennzeichnet, daß mit den Fokussiermotoren ein Brennpunktausgleichs-Steuergerät wirkungsverbunden ist, das zum Ausgleich von Sehschärfeunterschieden zwischen den Augen des Betrachters durch Feineinstellung der Scharfeinstellung der optischen Mittel dient.

9. Stereoskopisches Fernbetrachtungssystem nach Anspruch 1, dadurch gekennzeichnet, daß der Träger eine im wesentlichen halbkreisförmige Plattformführung (4) aufweist, ferner einen um eine im wesentlichen horizontale, sich längs eines Durchmessers der Führung erstreckende Achse (16) verschwenkbaren Führungsträger zum Höhenverstellen der Führung, und daß die Plattform (14) auf der Führung abgestützt und längs derselben bewegbar ist.

10. Stereoskopisches Fernbetrachtungssystem nach Anspruch 9, dadurch gekennzeichnet, daß zwischen der Plattform und der Führung ein Seitenrichtmotor (35) eingeschaltet ist, der dazu dient, zum Seitenrichten der Plattform diese längs der Führung zu bewegen, und daß zwischen der Führung und dem Führungsträger ein Höhenrichtmotor (20) eingeschaltet ist, der dazu dient, zum Höhenrichten der Plattform die Führung um die sich längs eines Durchmessers erstreckende Achse zu verschwenken.

11. Stereoskopisches Fernbetrachtungssystem nach Anspruch 10, dadurch gekennzeichnet, daß sich längs der Plattformführung (4) eine Zahnstange (33) erstreckt, daß der Seitenrichtmotor (35) auf dem Führungsträger montiert ist und eine Welle besitzt, und daß auf der Welle ein Schrägstirnrad (34) montiert ist, das mit der Zahnstange kämmt, so daß durch ein Drehen der Welle die Plattform längs der Plattformführung bewegt wird.

12. Stereoskopisches Fernbetrachtungssystem nach Anspruch 10, dadurch gekennzeichnet, daß an der Plattformführung (4) ein Ritzel befestigt ist, dessen Achse mit der sich längs eines Durchmessers der Führung erstreckenden Achse übereinstimmt, daß der Höhenrichtmotor (20) auf der Plattform montiert ist und eine Welle besitzt und daß auf der Welle ein mit dem Ritzel kämmendes Schrägstirnrad montiert ist, so daß durch ein Drehen der Welle die Plattformführung um die sich längs eines Durchmessers erstreckende Achse (16) verschwenkt wird.

13. Stereoskopisches Fernbetrachtungssystem nach Anspruch 1, dadurch gekennzeichnet, daß ein vom Träger der Kopfeinheit manuell steuerbares, robotartig bewegbares Werkzeug (110) vorgesehen ist, dessen Arbeitsvorgänge mit Hilfe des Betrachtungssystems betrachtbar sind.

14. Stereoskopisches Fernbetrachtungssystem nach Anspruch 1, gekennzeichnet durch eine im wesentlichen halbkreisförmige Plattformführung, durch einen die Plattformführung tragenden Plattformführungsträger zum Höhenrichten durch Verschwenken um eine sich längs eines Durchmessers der Führung erstreckende, horizontale Achse, wobei die Plattform in der Führung abgestützt und längs derselben bewegbar ist und Kamerasensoren trägt, durch einen zwischen der Plattform und der Führung eingeschalteten Seitenrichtmotor, der dazu dient, zum Seitenrichten der Plattform diese längs der Führung zu bewegen, durch einen zwischen der Führung und dem Führungsträger eingeschalteten Höhenrichtmotor, der dazu dient, zum Höhenrichten der Plattform die Führung um die sich längs eines Durchmessers erstreckende Achse zu verschwenken, und durch ein mit dem Seitenrichtmotor und dem Höhenrichtmotor verbundenes Kamerricht-Steuergerät das von einem das Betrachtungssystem benutzenden Betrachter zum Richten der Kameraplattform manipulierbar ist.

**Revendications**

1. Système stéréoscopique de vision éloignée comprenant des premier et second capteurs d'image (2, 3) espacés l'un de l'autre pour fournir un signal vidéo gauche et un signal vidéo droit, lesdits signaux vidéo représentant des scènes telles que saisies par lesdits capteurs d'image, une plateforme (14) portant lesdits capteurs (2, 3) pour faire converger sensiblement les axes de visée desdits capteurs sur le sujet à observer, un support déplaçable (4) pour déplacer ladite plateforme sélectivement dans les trois directions pour guider lesdits capteurs, et un moteur (20) pour déplacer ledit support de plateforme et une commande associée audit moteur, caractérisé par un ensemble frontal (7) prévu pour être porté sur la tête de l'observateur, un premier dispositif d'affichage vidéo (5) directement relié audit premier capteur d'image (2), un second dispositif d'affichage vidéo (6) directement relié audit second capteur d'image (3) respectivement pour recevoir lesdits signaux vidéo droit et gauche et des images affichées qui sont les reproductions des scènes saisies par lesdits capteurs d'image, lesdits premier et second dispositifs d'affichage vidéo étant montés sur ledit ensemble frontal et coopérant avec lesdits premier et second capteurs d'image pour fournir à l'observateur une image de vision stéréoscopique naturelle.

2. Système stéréoscopique de vision éloignée selon la revendication 1, caractérisé en ce qu'un ordinateur (10) est prévu pour le stockage et la recherche d'un texte ou d'une information graphique, et en ce que un sélecteur de sources vidéo (100) est prévu pour connecter sélectivement ledit dispositif d'affichage vidéo (5, 6) audit ordinateur (10) et auxdits capteurs d'image (2, 3).

3. Système stéréoscopique de vision éloignée selon la revendication 1, caractérisé en ce que les dispositifs d'affichage vidéo (5, 6) sont des tubes à rayons cathodiques, et en ce que l'ensemble frontal comprend des éléments optiques (68) disposés pour projeter indirectement sur les yeux dudit observateur les images reproduites sur lesdits tubes à rayons cathodiques.

4. Système stéréoscopique de vision éloignée selon la revendication 1, caractérisé en ce que chaque capteur d'image comprend un capteur d'image (38, 40), des moyens optiques (39, 41) disposés par rapport audit capteur d'image de façon à définir un axe de visée dudit capteur d'image, et un moteur de focalisation (44, 46) relié auxdits moyens optiques et destiné à placer lesdits moyens optiques de manière à focaliser une scène en un foyer le long dudit axe de visée dudit capteur d'image.

5. Système stéréoscopique de vision éloignée selon la revendication 4, caractérisé en ce que chaque capteur d'image comprend un moteur de zoom (45, 47) relié auxdits moyens optiques et destiné à faire varier la perspective optique de ladite scène telle que focalisée sur ledit capteur d'image.

6. Système stéréoscopique de vision éloignée selon la revendication 5, caractérisé en ce qu'un moteur de convergence (48, 49) est connecté entre le capteur d'image (2, 3) et la plateforme (14) pour déplacer le capteur d'image de manière à faire converger sensiblement les axes de visée dudit capteur d'image au foyer dudit capteur d'image.

7. Système stéréoscopique de vision éloignée selon la revendication 5, caractérisé en ce qu'une commande d'auto-focalisation (53) est connectée aux moteurs de focalisation (44, 46) et aux moteurs de zoom (45, 47) de manière à maintenir la focalisation desdits capteurs d'image pendant le fonctionnement desdits moteurs de zoom.

8. Système stéréoscopique de vision éloignée selon la revendication 4, caractérisé en ce qu'une commande d'équilibrage focal est en fonctionnement reliée aux moteurs de focalisation pour le réglage fin des positions focalisation des moyens optiques de manière à compenser les acuités visuelles individuelles des yeux dudit observateur.

9. Système stéréoscopique de vision éloignée selon la revendication 1, caractérisé en ce que le support comprend une piste de plateforme sensiblement semi-circulaire (4), un support de piste supportant ladite piste de plateforme pour faire monter ladite piste par pivotement autour d'un axe diamétral sensiblement horizontal (16), et en ce que la plateforme (14) est portée sur ladite piste de manière à être déplacée le long de ladite piste.

10. Système stéréoscopique de vision éloignée selon la revendication 9, caractérisé en ce qu'un moteur azimutal (35) est disposé entre la plateforme et la piste destinée à déplacer lesdits moyens de plateforme le long de ladite piste de manière à provoquer un mouvement azimutal de ladite plateforme, et en ce qu'un moteur d'élévation (20) est disposé entre ladite piste et ledit support de piste de manière à faire pivoter ladite piste autour d'un axe diamétral pour provoquer la montée.

11. Système stéréoscopique de vision éloignée selon la revendication 10, caractérisé en ce qu'une crémaillère (33) s'étend le long de ladite piste de plateforme (4), en ce que le moteur azimutal (35) est monté sur ledit support de piste et disposé d'un arbre tournant, et en ce que un pignon à pas hélicoïdal (34) est disposé sur ledit arbre et coopère avec ladite crémaillère lorsque la rotation dudit arbre entraîne ladite plateforme le long de ladite piste de plateforme.

12. Système stéréoscopique de vision éloignée selon la revendication 10, caractérisé en ce qu'un pignon est fixé sur ladite piste de plateforme (4), ledit pignon ayant un axe disposé coaxialement avec ledit axe diamétral de ladite piste, en ce que le moteur d'élévation (20) est monté sur ladite plateforme et disposé d'un arbre tournant, et en ce qu'un pignon à pas hélicoïdal est disposé sur ledit arbre et coopère avec le pignon lorsque la rotation dudit arbre provoque le pivotement de ladite piste de plateforme autour dudit axe diamétral (16).

13. Système stéréoscopique de vision éloignée selon la revendication 1, caractérisé en ce que un robot (110) est commandable manuellement par un levier dudit ensemble frontal pour visualiser à 'aide dudit système de vision les opérations effectuées par ledit robot.

14. Système stéréoscopique de vision éloignée selon la revendication 1, caractérisé par une piste de plateforme sensiblement semi-circulaire, un support de piste de plateforme portant ladite piste de plateforme pour l'élévation par pivotement autour d'un axe diamétral horizontal de ladite piste, et une plateforme étant portée dans ladite piste pour se déplacer le long de ladite piste et portant des capteurs de caméra, un moteur azimutal disposé entre ladite plateforme et ladite piste destinée à déplacer ladite plateforme le long de ladite piste de manière à faire pivoter ladite piste autour dudit axe diamétral afin de provoquer l'élévation de ladite plateforme, et des moyens de commande de la caméra connectés auxdits moteurs azimutal et d'élévation destinés à être manipulés par un observateur utilisant ledit système de vision pour guider ladite plateforme de caméra.

Fig.1.

EP 0 218 751 B1

*Fig.2.*

*Fig.3.*

*Fig.4.*

Fig.5.

Fig.6.

Fig.7.

Fig.8.

EP 0 218 751 B1

# Fig.9.

EP 0 218 751 B1